# EUROPÄISCHE PATENTSCHRIFT

(11) **EP 2 554 922 B1**
(45) Veröffentlichungstag und Bekanntmachung des Hinweises auf die Patenterteilung: **04.09.2013**
(21) Anmeldenummer: 12191013.7
(22) Anmeldetag: 15.04.2011
(51) Int. Cl.: F24H 1/12, F24H 1/14, H05B 3/12, F24H 9/18, A61M 16/16, A61M 11/04

(54) **Verdampfer sowie Verfahren zum Betreiben eines solchen Verdampfers**
Evaporator and method for operating such an evaporator
Évaporateur et procédé de fonctionnement d'un tel évaporateur

(30) Priorität: 04.05.2010 DE 102010016783
(43) Veröffentlichungstag der Anmeldung: 06.02.2013
(62) Teilanmeldung aus: 11162532.3
(73) Patentinhaber: WIK Far East Ltd, North Point Hong Kong (CN)
(72) Erfinder: Kock, Marwin, 45239 Essen (DE)
(74) Vertreter: Haverkamp, Jens

(56) Entgegenhaltungen:
- WO-A1-2010/025955
- WO-A2-2004/038303
- DE-A1- 2 845 894
- DE-U1-202008 003 357
- GB-A- 2 305 233
- US-A- 4 343 988
- US-A- 4 371 777
- US-B1- 6 330 395

## Beschreibung

Die Erfindung betrifft einen Verdampfer zum Verdampfen einer Flüssigkeit, welcher Verdampfer insbesondere Teil eines Vaporisators zum Applizieren von Dampf an ein menschliches Körperteil ist und welcher Verdampfer aus mehreren Teilen zusammengesetzt ist und über ein PTC-Heizelement verfügt, welches PTC-Heizelement zwischen zwei das PTC-Heizelement einfassenden Aluminiumblockteilen angeordnet ist, welche Blockteile außenseitig über einen sich mäandrierend über die Längserstreckung des Blockteils erstreckenden Kanal verfügen. Ferner betrifft die Erfindung ein Verfahren zum Betreiben eines solchen Verdampfers.

Für die äußerliche Behandlung von menschlichen Körperteilen, beispielsweise der Füße, der Hände oder des Gesichtes einer Person, werden Bäder verwendet, bei Füßen Fußbäder. Derartige Fußbäder verfügen über eine mit der Behandlungsflüssigkeit befüllbare Wanne und hinreichend Raum, dass die Füße einer Person darin einsetzbar sind. Typischerweise umfasst ein solches Fußband eine Heizeinrichtung zum Erwärmen des Wassers. Zumeist verfügen derartige Fußbäder zudem über Einrichtungen, mit denen ein Fuß massiert werden kann. Je nach der gewünschten Massagetätigkeit, befinden sich diese Einrichtungen innerhalb der Wanne oder in einem die beiden Fußbereiche der Wanne trennende Mittelsteg oberhalb des Flüssigkeitsspiegels. Bekannt ist ferner, in das Bad Luft- oder Wasserströme einzudüsen, um den Effekt eines Whirlpools zu erreichen.

Auch wenn sich mit einem solchermaßen konzipierten Behandlungsgerät unterschiedliche Anwendungen und Behandlungen realisieren lassen, wäre es wünschenswert, wenn ebenfalls Behandlungsgeräte bekannt wären, mit denen menschliche Körperteile, beispielsweise die Füße, mit feuchtem Dampf behandelt werden könnten. Hierbei ist zu berücksichtigen, dass eine solche Behandlung naturgemäß nicht mit Heißdampf erfolgen kann, was zu Verbrühungen führen würde.

Für derartige Behandlungsgeräte werden Verdampfer zum Verdampfen der Behandlungsflüssigkeit benötigt.

Ein Verdampfer mit den Merkmalen des Obergriffs des Anspruchs 1 ist aus WO 2004/038303 A2 bekannt. WO 2004/038303 A2 offenbart einen Verdampfer mit einem Sandwichaufbau. Aufgenommen zwischen zwei flachen Blockteilen, in denen jeweils ein Fluidkanal enthalten ist, ist eine PTC-Heizelementanordnung. Bei den Blockteilen handelt es sich um Gussstücke aus einem gut Wärme leitenden Material, in welches der Fluidkanal in Form eines Rohres eingegossen ist. Zwischen den beiden Blöcken ist eine PTC-Heizelementanordnung angeordnet, die in Wärme leitender Verbindung an die zueinander weisenden Seiten der Blockteile anliegt. Zwischen den beiden Blockteilen besteht eine Fluidverbindung in Form eines außerhalb der Blockteile angeordneten Verbindungsrohrstücks. An der Eingangsseite des Verdampfer befindet sich ein Steuerventil zum Steuern der dem Heizelement zugeführten Flüssigkeitsmenge.

Dem Gegenstand der vorliegenden Erfindung liegt somit die Aufgabe zu Grunde, eine gleichmäßige Dampfströmung zu gewehrleisten.

Gelöst wird diese Aufgabe durch einen eingangs genannten, gattungsgemäßen Verdampfer, bei dem die Kanäle außenseitig in den Aluminiumblockteilen verlaufen und jeweils durch einen Deckel verschlossen sind, wobei sich in dem einem Deckel eine Zuströmöffnung und in dem anderen Deckel ein Ausgang befinden, und bei dem der Querschnitt des Kanals des Blockteils, welches mit dem den Ausgang aufweisenden Deckel verschlossen ist, kleiner bemessen ist als der Querschnitt des Kanals des anderen Blockteils.

Der Verdampfer ist mittels eines PTC-Heizelementes betrieben. Ausgenutzt wird bei diesem Konzept die Leistungscharakteristik eines solchen Heizelementes, da dieses hinsichtlich seiner Zieltemperatur eingestellt bzw. in Bezug auf eine bestimmte Zieltemperatur ausgeführt sein kann. Zudem ist die Heizkurve eines solchen PTC-Heizelementes von seiner aktuellen Temperatur abhängig. Daher kann ein solches Heizelement länger in einem Temperaturbereich gehalten werden, in dem ein Dampf mit besonders hoher Feuchtigkeitstragung erzeugt werden kann. Die Temperatur für einen solchen Dampf liegt bei etwa 135 Grad Celsius. Aufgrund der Trägheit eines PTC-Heizelementes kann die Zufuhr von zu verdampfendem Wasser in Abhängigkeit von der aktuellen Temperatur des PTC-Heizelementes gesteuert werden. Bei einer solchen Ausgestaltung wird zu verdunstendes Wasser pulsierend dem Verdampfer zugeführt. Bei dieser Betriebsweise kann der Verdampfer zwischen einer Temperatur von 130 Grad Celsius und 140 Grad Celsius durch entsprechende Wasserzuführung gehalten werden. Insofern ist der Einsatz eines oder auch mehrerer PTC-Heizelemente zum Erzeugen des gewünschten Dampfes zweckmäßig.

Weitere Vorteile und Ausgestaltungen der Erfindung ergeben sich aus der nachfolgenden Beschreibung eines Ausführungsbeispiels unter Bezugnahme auf die beigefügte Figur. Es zeigen:
- **Fig. 1:**: eine schematisierte Darstellung eines Vaporisators gemäß einem ersten Ausführungsbeispiel,
- **Fig. 2:**: eine schematisierte Darstellung eines Vaporisators gemäß einem weiteren Ausführungsbeispiel und
- **Fig. 3:**: eine schematisierte Schnittdarstellung eines Verdampfers.

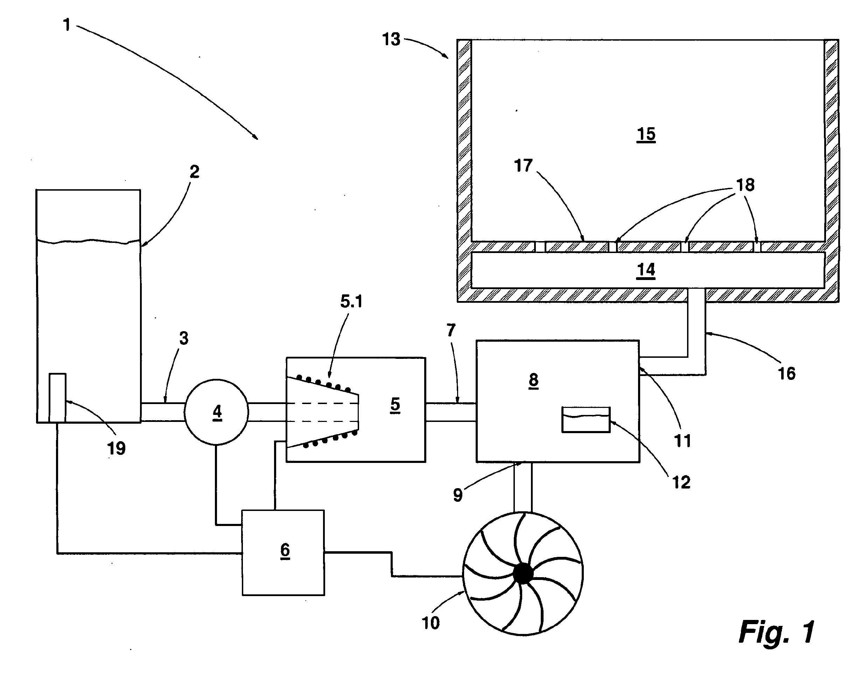

Figur 1 zeigt einen Vaporisator 1 zum Applizieren eines Niedrigtemperaturdampfes an ein menschliches Körperteil in einer schematisierten Darstellung. In natura ist der Vaporisator 1 bei diesem Ausführungsbeispiel in ein herkömmliches Fußbad integriert. Somit verfügt das Fußbad neben den ansonsten üblichen Aggregaten zudem über die dem Vaporisator 1 zugehörigen Elemente.

Der Vaporisator 1 umfasst ein Vorratsbehältnis 2 zum Bevorraten von Wasser, welches bei diesem Ausführungsbeispiel als Behandlungsflüssigkeit verwendet wird. Das Vorratsbehältnis 2 ist ein innerhalb des Fußbades befindlicher Tank. Der Ausgang des Vorratsbehältnisses 2 ist an eine Förderleitung 3 angeschlossen, über die Wasser aus dem Vorratsbehältnis 2 durch eine bei dem dargestellten Ausführungsbeispiel elektromotorisch betriebene Pumpe 4 einem in einer Verdampferkammer 5 angeordneten Verdampfer 5.1 zugeführt wird. Die Pumpe 4 und der Verdampfer 5 sind hinsichtlich ihrer Leistung regulierbar und zu diesem Zwecke an eine Steuereinheit 6 angeschlossen. Mithin wird über die Steuereinheit 6 die Leistungsaufnahme der Pumpe 4 sowie des Verdampfers 5 angesteuert. Damit kann die Pumpe auch pulsierend angesteuert werden. In nicht näher dargestellter Art und Weise ist an die Steuereinheit 6 ebenfalls ein Stellglied angeschlossen, mit dem ein Benutzer den Vaporisator ein- und ausschalten kann und mit dem ein Benutzer bestimmte Dampfparameter, beispielsweise Temperatur und Feuchtigkeitsgehalt, einstellen kann.

Der Ausgang des Verdampfers 5 ist über eine Förderleitung 7 an eine Mischkammer 8 angeschlossen. Somit wird der durch den Verdampfer 5 bereitgestellte Heißdampf als Heißdampfstrom über die Förderleitung 7 in die Mischkammer 8 gefördert. In die Mischkammer 8 mündet des Weiteren der Ausgang 9 eines Gebläses 10, welches bei dem dargestellten Ausführungsbeispiel als Förderglied einen Ventilator umfasst. Über das Gebläse 10 wird auf Umgebungstemperatur befindliche Umgebungsluft in die Mischkammer 8 eingebracht. Die hinsichtlich ihrer Temperatur in die Mischkammer 8 zugeführte Umgebungsluft vermischt sich mit dem Heißdampfstrom und kühlt diesen in Abhängigkeit von der Temperaturdifferenz zwischen dem Heißdampfstrom und dem zugeführten Umgebungsluftstrom und dem Mischungsverhältnis, mit dem diese beiden Ströme zugeführt werden, ab. Das Mischungsverhältnis wird eingestellt, damit am Ausgang 11 der Mischkammer ein Niedrigtemperaturdampfstrom austritt. Die Temperatur entspricht der Behandlungstemperatur. Gleichzeitig wird das Verhältnis der Mischung von Heißdampfstrom und zugeführter Umgebungsluft so eingestellt, dass die durch den Heißdampfstrom in die Mischkammer 8 eingebrachte Feuchtigkeit nicht kondensiert. Mithin bleibt der Feuchtigkeitsgehalt des Heißdampfes zumindest im Wesentlichen erhalten. Daher ist im Gegensatz zur weitestgehenden Sättigung des Heißdampfstromes hinsichtlich seiner Wasseraufnahmekapazität der über das Gebläse 10 zugeführte kühlende Umgebungsluftstrom hinsichtlich seiner Wasseraufnahmekapazität ungesättigt.

Innerhalb der Mischkammer 8 befindet sich ein Verdunster 12. Der Verdunster 12 ist in nicht dargestellter Art und Weise in die Mischkammer 8 durch Öffnen einer Klappe einsetzbar und aus dieser herausnehmbar. Der Verdunster 12 dient zur Aufnahme von Behandlungsstoffen, die mit dem Niedrigtemperaturdampf über den Ausgang 11 dem zu behandelnden Körperteil, in diesem Fall den Füßen, zugeführt werden soll. Bei den Behandlungsstoffen kann es sich beispielsweise um ätherische Öle handeln.

Dem Ausgang 11 der Mischkammer 8 nachgeschaltet ist das eigentliche Behandlungsgefäß 13. Dieses ist in einen Entspannungsraum 14 und einen Nutzraum 15 unterteilt. In dem Entspannungsraum 14 wird der über eine Leitung 16 zugeführte feuchtigkeitsbeladene Niedrigtemperaturdampf über eine gewisse Fläche verteilt. Mithin dient der Entspannungsraum 14 als Ausgangssammler. In die den Entspannungsraum 14 von dem Nutzraum 15 trennende Wand 17, die bei dem dargestellten Ausführungsbeispiel die Bodenwand des Nutzraumes 15 ist, sind mehrere Durchbrechungen 18 eingebracht, durch die der Niedrigtemperaturdampf in den Nutzraum 15 eintritt. Die Durchbrechungen 18 sind über den Boden 17 oder auch in der Seitenwand des Nutzraumes 15 verteilt, damit die in das Fußbad eingesetzten Füße aus unterschiedlichen Richtungen dampfbeaufschlagt sind.

Verfügt das Fußbad neben der vorbeschriebenen Vaporisatoreinrichtung auch über eine Einrichtung zum Zuführen von Luft in den Nutzraum 15, wenn dieser wasserbefüllt ist, kann das Gebläse 10 gleichfalls dem Zweck des Zuführens von Luft in den Nutzraum dienen. Eine solchermaßen vorgesehene Luftzufuhr wird über eine andere Wirksamkeit realisiert. Dann befindet sich in der Verbindung zwischen dem Gebläse 10 und der Mischkammer 8 ein Ventil, welches bei einem solchermaßen vorgesehenen Betrieb geschlossen ist. Das Gebläse fördert dann die in den Nutzraum über Düsen einzubringende Luft über eine weitere Luftzuführleitung (in der Figur nicht dargestellt). Das Gebläse 10 mit seinem Ventilator ist leistungsregulierbar und zu diesem Zweck an die Steuereinheit 6 angeschlossen.

Das Vorratsbehältnis 2 verfügt über einen Füllstandssensor 19, der ebenfalls von der Steuereinheit 6 auslesbar ist. Ist der Füllstand innerhalb des Vorratsbehältnisses 2 zu gering oder fällt dieser unter eine vorbeschriebene Mindestmenge ab, können aus Sicherheitsgründen die Pumpe 4 und der Verdampfer 5 ausgeschaltet werden. Gleichzeitig wird ein Signal generiert, mit dem einem Benutzer signalisiert wird, dass Wasser in das Vorratsbehältnis 2 nachzufüllen ist.

Die Steuereinheit 6 steuert in Abhängigkeit von einem vorgegebenen Steueralgorithmus und einer nutzerseitigen Einstellung den Betrieb des Vaporisators 1 bzw. seiner Aktoren - der Pumpe 4, des Verdampfers 5 und des Gebläses 10. Über die Leistungsregulierung der Pumpe 4 und des Verdampfers 5 ist die generierte Heißdampfmenge und die Temperatur des generierten Heißdampfes einstellbar. Die Leistungsregelbarkeit des Gebläses 10 erlaubt das Mischungsverhältnis zwischen dem über die Förderleitung 7 strömenden Heißdampfstrom und dem über das Gebläse 10 zugeführten kühlenden Umgebungsluftstromes einzustellen. Gleichermaßen kann dieses Mischungsverhältnis auch durch entsprechende Ansteuerung der Pumpe 4 sowie des Verdampfers 5 beeinflusst werden.

Bei einer Benutzung eines solchermaßen konzipierten Fußbades kann die Applikation von Niedrigtemperaturdampf, der sich auf einer für einen Benutzer angenehmen Temperatur befindet und daher als "warm" wahrgenommen wird, mit einer über Düsen in den Nutzraum 15 zugeführten Kaltwasser (nicht dargestellt) abwechseln. Ein solche wiederholte abwechselnde Behandlung der Füße mit Niedrigtemperaturdampf und Kaltwasser entspricht einer so genannten Kneipp'schen Anwendung.

In dem Entspannungsraum kondensierte Flüssigkeit kann über eine nicht dargestellte Abzugseinrichtung entfernt werden.

Das Vorsehen des Gebläses 10 bei dem Vaporisator 1 kann ferner dazu genutzt werden, dieses für die Zwecke der Trocknung der Dampfwegsamkeiten einzusetzen. Somit können durch einfachen Nachlauf des Gebläses 10 nach Abschalten der Pumpe 4 und des Verdampfers 5 in der Mischkammer 8 befindliche Feuchtigkeitsreste durch den durch das Gebläse 10 geförderten Luftstrom aufgenommen und über die Leitung 16, den Entspannungsraum 14 und den Nutzraum 15 entfernt werden. Im Zuge eines solchermaßen geförderten Luftstromes wird gleichzeitig die Leitung 16, der Entspannungsraum 14 und der Nutzraum 15 getrocknet. Somit kann durch Betrieb des Gebläses 10 das Gerät nach einem vorzugsweise vorherigem Auswischen desselben vollständig getrocknet werden.

Ist eine Desinfektion gewünscht, kann diesem Luftstrom über eine typischerweise in die Mischkammer 8 mündende Einspritzvorrichtung ein Desinfektionsmittel beigemengt werden.

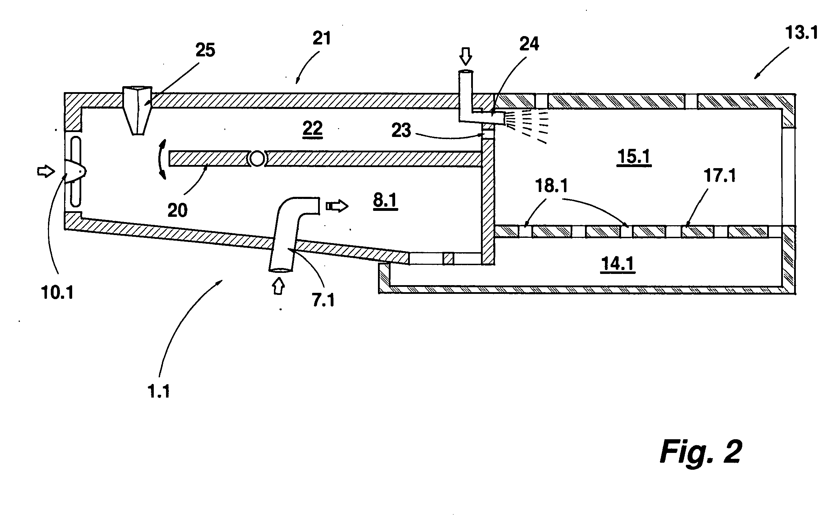

Figur 2 zeigt einen Vaporisator 1.1 gemäß einem weiteren Ausführungsbeispiel. Der Vaporisator 1.1 arbeitet nach demselben Prinzip zum Applizieren eines Niedrigtemperaturdampfes wie dieses zu dem Vaporisator 1 der Figur 1 beschrieben ist. Daher gelten die diesbezüglichen Ausführungen auch für den Vaporisator 1.1.

Der Vaporisator 1.1 verfügt ebenfalls über ein Behandlungsgefäß 13.1. Unterhalb des auch als Behandlungsraum angesprochenen Nutzraumes 15.1 des Behandlungsgefäßes 13.1 befindet sich ein Entspannungsraum 14.1, der ebenso wie bei dem Ausführungsbeispiel der Figur 1 zur Dampfverteilung dient. Der Nutzraum 15.1 ist von dem Entspannungsraum 14.1 durch eine Durchbrechungen 18.1 aufweisende Wand 17.1 getrennt. In den Entspannungsraum 14.1 mündet der Ausgang einer Mischkammer 8.1. Die Mischkammer 8.1 des Vaporisators 1.1 vergrößert sich in Richtung zu dem Ausgang zum Entspannungsraum 14.1 hin. In die Mischkammer 8.1 mündet eine Förderleitung 7.1, durch die von einem Verdampfer erzeugter Heißdampf, wie durch die Pfeile schematisiert dargestellt, zugeführt wird. Die Förderleitung 7.1 ist innerhalb der Mischkammer 8.1 in Strömungsrichtung zum Entspannungsraum 14.1 hin abgewinkelt. Die Mischkammer 8.1 ist an ihrem dem Ausgang zum Entspannungsraum 14.1 hin gegenüber liegenden Ende durch einen nicht dargestellten Aktor ansteuerbare Stellklappe 20 begrenzt. Das Gebläse 10.1 bei dem Vaporisator 1.1 sitzt an einer Stirnseite des sich an den Nutzraum 15.1 anschließenden Gehäuseteils 21. Selbstverständlich kann das Gebläse 10.1 auch außerhalb sitzen; dann mündet die Luftförderleitung vorzugsweise an der in Figur 2 gezeigten Stelle in das Gehäuseinnere. Oberhalb der Mischkammer 8.1 befindet sich ein Luftkanal 22. Der Luftkanal 22 kann ebenso wie die Mischkammer 8.1 durch die Stellklappe 20 verschlossen werden. Somit kann bei einem Betrieb des Gebläses 10.1 in Abhängigkeit von der Stellung der Stellklappe 20 die geförderte Luft entweder in die Mischkammer 8.1 oder in den Luftkanal 22 eingebracht werden. Es versteht sich, dass auch ein Mischbetrieb, bei dem Luft sowohl der Mischkammer 8.1 als auch dem Luftkanal 22 zugeführt wird, denkbar ist. Der Luftkanal 22 mündet bei dem dargestellten Ausführungsbeispiel in den oberen Bereich des Nutzraumes 15.1.

Oberhalb der Mündung 23 des Luftkanals 22 in den Nutzraum 15.1 ist der Ausgang einer Kaltwassersprüheinrichtung 24 angeordnet. Über diese kann Kaltwasser in den Nutzraum 15.1 eingesprüht werden.

In Strömungsrichtung der durch das Gebläse 10.1 geförderten Luft der Stellklappe 20 vorgeschaltet ist in die obere Wand des Gehäuseteils 21 eine Kartusche 25, bei dem dargestellten Ausführungsbeispiel aus Sintermetall, in das Gehäuseteilinnere eingreifend angeordnet. Die Kartusche 25 dient zur Aufnahme von Duft- und/oder Pflegestoffen, die bei einem Betrieb des Vaporisators 1.1 an die durch das Gebläse 10.1 geförderte Luft abgegeben und sodann in den Nutzraum 15.1 transportiert werden.

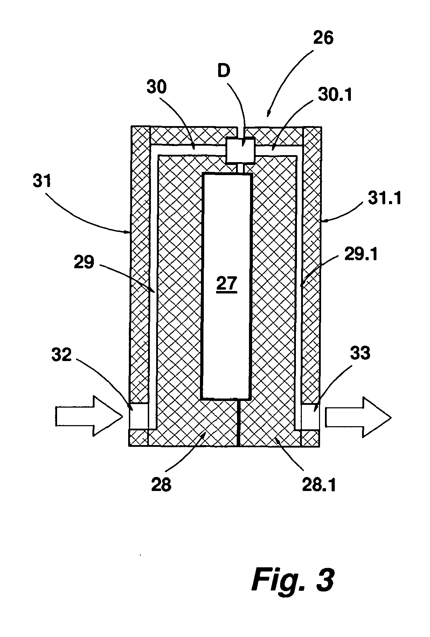

Figur 3 zeigt einen Verdampfer 26, dessen Kern ein PTC-Heizelement 27 ist. Das PTC-Heizelement 27 ist eingebettet in ein Strömungswegsamkeiten enthaltenden Aluminiumblock. Der Aluminiumblock des dargestellten Ausführungsbeispiels ist aus mehreren Teilen zusammengesetzt. Unmittelbar das PTC-Heizelement 27 einfassend sind zwei Blockteile 28, 28.1 vorgesehen. Zwischen diesen ist das PTC-Heizelement 27 unter einer gewissen Vorspannung stehend mit seinen Flachseiten gehalten. Dieses dient zum Bereitstellen eines besonders guten Wärmeüberganges von dem PTC-Heizelement 27 auf die Blockteile 28, 28.1. Das Blockteil 28 verfügt außenseitig über eine sich mäandrierend über die Längserstreckung des Blockteils 28 erstreckenden Kanal 29, der an seinem in Strömungsrichtung befindlichen Ende in einen Verbindungskanal 30 übergeht. Das Blockteil 28.1 ist prinzipiell gleich aufgebaut, jedoch strömt in diesem das Fluid von dem Verbindungskanal 30.1 in den in gleicher Weise mäandrierend konzipierten Strömungskanal 29.1. Die beiden Verbindungskanäle 30, 30.1 sind durch eine Dichtungshülse D miteinander verbunden. Die Dichtungshülse D ist eingesetzt in eine entsprechende Hülsenausnehmung in jeweils einem Blockteil 28 bzw. 28.1. Die Strömungskanäle 29, 29.1 sind durch Fräsen in die jeweiligen Blockteile 28, 28.1 eingebracht. Verschlossen sind diese Kanäle 29, 29.1 jeweils durch einen Deckel 31, 31.1. In dem Deckel 31 befindet sich eine Zuströmöffnung 32. In dem Deckel 31.1 ein Ausgang 33. Durch den Zustromeingang 32 wird Wasser an den Verdampfer 26 geführt. Aus dem Ausgang 33 tritt Dampf aus, wie dieses schematisiert durch die Blockpfeile in Figur 3 dargestellt ist.

Bei einem Betrieb des Verdampfers 26 wird durch den Eingang 32 Wasser in den Verdampfer 26 eingebracht, verdampft an dem auf entsprechender Temperatur befindlichen Aluminium, tritt durch den Ausgang 33 aus und wird sodann beispielsweise über die Förderleitung 7.1 der Mischkammer 8.1 zugeführt. Im Zuge des Verdampfens wird den Aluminiumblockteilen 28, 28.1 Wärme entzogen. Gleiches gilt sodann für das PTC-Heizelement 27, welches bei entsprechender Wasserbeaufschlagung der Strömungswegsamkeit ebenfalls abgekühlt wird. Durch die Trägheit des PTC-Heizelementes 27 benötigt dieses somit gewisse Ruhezeiten, in denen durch die Aluminiumblockteile 28, 28.1 kein Wasser für die Dampferzeugung hindurchgepumpt wird, damit das PTC-Heizelement 27 wieder auf die vorgesehene Temperatur erwärmt ist. Daher wird man den Verdampfer 26 typischerweise mit einer gepulsten Wasserzuführung steuern, und zwar abhängig von der Temperatur des PTC-Heizelementes.

Für die Zwecke des Bereitstellens einer gleichmäßigen Dampfströmung ist der Querschnitt der Fluidkanäle 29.1 geringfügig kleiner bemessen als derjenige der Strömungskanäle 29. Dadurch wird ein gewisser Staudruck erzeugt mit der Folge, dass der erzeugte Dampf aus dem Ausgang 33 gleichmäßig ausströmt. Bei einem gepulsten Betrieb des Verdampfers 26 trifft das gleichmäßige Ausströmen des Dampfes für die wasserbeaufschlagten Pulszeiten zu.

Die elektrischen/elektronischen Aktoren des Vaporisators 1.1 sind in nicht näher dargestellter Art und Weise an einer Steuereinrichtung angeschlossen und können mithin von dieser angesteuert werden. Aufgrund der unterschiedlichen Möglichkeiten einer Behandlung mit dem Vaporisator 1.1 können von einem Nutzer vorbestimmte Programme ausgewählt werden, die sodann von der Steuereinheit abgearbeitet werden. Hierbei kann es sich um eine reine Dampfbehandlung handeln. Dann befindet sich die Stellklappe 20 in ihrer den Luftkanal 22 verschließenden Stellung. Ebenfalls ist eine Behandlung ohne Dampf möglich. Dann befindet sich die Stellklappe 20 in ihrer den Mischraum 8.1 verschließenden Stellung. Auch ist ein Wechselprogramm möglich, bei dem für eine vorbestimmte Zeit der durch das Gebläse 10.1 generierte Luftstrom durch die Mischkammer 8.1 und entsprechender Dampfzuführung geleitet wird und wechselweise durch den Luftkanal 22. In gleicher Weise kann die Kaltwassersprüheinrichtung ebenfalls Teil eines Programms sein. Auch eine manuelle Steuerung der Kaltwassersprüheinrichtung 24 ist möglich.

Aufgrund der besonders hohen Feuchtigkeitstragung des Dampfes in Form vieler kleiner Wassertröpfchen eignen sich die vorbeschrieben Vaporisatoren bzw. die Behandlungsgefäße besonders, um den darin befindlichen Dampf zu illuminieren, beispielsweise mit einer LED-Beleuchtungseinrichtung. Eine solche Illumination kann farbig auch in Abhängigkeit von dem jeweiligen Behandlungsprogramm oder dem aktuell durchgeführten Behandlungsschritt unterschiedlich farbig ausgeführt sein.

In einer Weiterbildung ist vorgesehen, dem Behandlungsgefäß einen oder mehrere Aufsätze, typischerweise als Deckelaufsätze konzipiert, zuzuordnen, um eine Behandlung unterschiedlicher Körperteile in Abhängigkeit von dem jeweils gewählten Aufsatz zu ermöglichen. So kann beispielsweise ein Gesichtsaufsatz vorgesehen sein, dessen obere Kante an die Kontur des Gesichtes einer Person angepasst ist. Zusätzlich kann bei einer solchen Behandlung der Vaporisator auf ein Podest aufgestellt sein, mit dem dieser für eine ergonomisch günstigere Behandlung geneigt ist.

Die Beschreibung der Erfindung verdeutlicht, dass mit dem beschriebenen Konzept des Vaporisators und dem beschriebenen Verfahren sich ein solcher Vaporisator auch mit geringem Bauvolumen realisieren lässt. Aus diesem Grunde eignet sich diese Konzeption vor allem auch dann, wenn eine Vaporisatorfunktion in ein bestehendes Gerät, wie beispielsweise ein Fußbad oder dergleichen zusätzlich integriert werden soll.

### Bezugszeichenliste

| | |
|---|---|
| 1, 1.1 | Vaporisator |
| 2 | Vorratsbehältnis |
| 3 | Förderleitung |
| 4 | Pumpe |
| 5 | Verdampferkammer |
| 5.1 | Verdampfer |
| 6 | Steuereinheit |
| 7, 7.1 | Förderleitung |
| 8, 8.1 | Mischkammer |
| 9 | Ausgang |
| 10, 10.1 | Gebläse |
| 11 | Ausgang |
| 12 | Verdunster |
| 13, 13.1 | Behandlungsgefäß |
| 14, 14.1 | Entspannungsraum |
| 15, 15.1 | Nutzraum |
| 16 | Leitung |
| 17, 17.1 | Wand |
| 18, 18.1 | Durchbrechung |
| 19 | Füllstandssensor |
| 20 | Stellklappe |
| 21 | Gehäuseteil |
| 22 | Luftkanal |
| 23 | Mündung |
| 24 | Kaltwassersprüheinrichtung |
| 25 | Kartusche |
| 26 | Verdampfer |
| 27 | PTC-Heizelement |
| 28, 28.1 | Aluminiumblockteil |
| 29, 29.1 | Fluidkanal |
| 30, 30.1 | Verbindungskanal |
| 31, 31.1 | Deckel |
| 32 | Eingang |
| 33 | Ausgang |
| D | Dichtungshülse |

## Patentansprüche

1. Verdampfer zum Verdampfen einer Flüssigkeit, welcher Verdampfer insbesondere Teil eines Vaporisators (1, 1.1) zum Applizieren von Dampf an ein menschliches Körperteil ist, wobei der Verdampfer (26) aus mehreren Teilen (28, 28.1, 31, 31.1) zusammengesetzt ist und über ein PTC-Heizelement (27) verfügt, welches PTC-Heizelement (27) zwischen zwei das PTC-Heizelement (27) einfassenden Aluminiumblockteilen (28, 28.1) angeordnet ist, welche Blockteile (28, 28.1) außenseitig über einen sich mäandrierend über die Längserstreckung des Blockteils (28, 28.1) erstreckenden Kanal (29, 29.1) verfügen, **dadurch gekennzeichnet, dass** die Kanäle (29, 29.1) außenseitig in den Blockteilen (28, 28.1) verlaufen und jeweils durch einen Deckel (31, 31.1) verschlossen sind, wobei sich in dem einem Deckel (31, 31.1) eine Zuströmöffnung (32) und in dem anderen Deckel ein Ausgang (33) befinden, und dass der Querschnitt des Kanals (29.1) des Blockteils (28.1), welches mit dem den Ausgang (33) aufweisenden Deckel (31.1) verschlossen ist, kleiner bemessen ist als der Querschnitt des Kanals (29) des anderen Blockteils (28).

2. Verdampfer nach Anspruch 1, **dadurch gekennzeichnet, dass** das PTC-Heizelement (27) zwischen den Blockteilen (28, 28.1) unter Vorspannung stehend mit seinen Flachseiten gehalten ist.

3. Verdampfer nach Anspruch 1 oder 2, **dadurch gekennzeichnet, dass** jedes Blockteil (28, 28.1) einen Verbindungskanal (30, 30.1) aufweist, die miteinander durch eine Dichtungshülse D verbunden sind.

4. Verfahren zum Bereitstellen einer gleichmäßigen Dampfströmung mit einem Verdampfer nach einem der Ansprüche 1 bis 3, **dadurch gekennzeichnet, dass** eine Wasserzufuhr an den Verdampfer (26) gepulst erfolgt.

5. Verfahren nach Anspruch 4, **dadurch gekennzeichnet**, die gepulste Wasserzuführung abhängig von der aktuellen Temperatur des PTC-Heizelementes (27) durchgeführt wird.

## Claims

1. An evaporator for evaporating a liquid, which evaporator is in particular part of a vaporiser (1, 1.1) for the applying of steam to a human body part, with the evaporator (26) being composed of a plurality of parts (28, 28.1, 31, 31.1) and having a PTC heating element (27), which PTC heating element (27) is disposed between two aluminium block parts (28, 28.1) enclosing the PTC heating element (27), which block parts (28, 28.1) exhibit on the outside a meandering channel (29, 29.1) extending over the longitudinal extension of the block part (28, 28,1), **characterised in that** the channels (29, 29.1) run in the block parts (28, 28.1) on the outside and are each closed by a cover (31, 31.1), there being an inlet opening (32) in the one cover (31, 31 .1) and in the other cover an outlet (33), and wherein the cross-section of the channel (29.1) of the block part (28.1) which is closed with the cover (31.1) exhibiting the outlet (33) is smaller in dimension than the cross-section of the channel (29) of the other block part (28).

2. The evaporator of claim 1 **characterised in that** the PTC heating element (27) is held between the block parts (28, 28.1) under preload upright by its flat sides.

3. The evaporator of claim 1 or 2 **characterised in that** each block part (28, 28.1) exhibits a connection channel (30, 30.1) which are interconnected by a sealing sleeve D.

4. A method for providing an even flow of steam with an evaporator according to any one of claims 1 to 3, **characterised in that** a supply of water to the evaporator (26) is effected in a pulsed manner.

5. The method of claim 4 **characterised in that** the pulsed water supply is executed out depending on the current temperature of the PTC heating element (27).

## Revendications

1. Évaporateur destiné à évaporer un liquide, lequel évaporateur fait notamment partie d'un vaporisateur (1, 1.1) destiné à appliquer de la vapeur sur une partie d'un corps humain, l'évaporateur (26) étant composé de plusieurs pièces (28, 28.1, 31, 31.1) et disposant d'un élément chauffant PTC (27), lequel élément chauffant PTC (27) est disposé entre deux blocs en aluminium (28, 28.1) entourant l'élément chauffant PTC (27), lesquels blocs (28, 28.1) disposent vers l'extérieur d'un canal (29, 29.1) qui parcourt, en méandres, l'étendue longitudinale du bloc (28, 28.1), **caractérisé en ce que** les canaux (29, 29.1) s'étendent vers l'extérieur dans les blocs (28, 28.1) et qu'ils sont respectivement fermés par un couvercle (31, 31.1), une ouverture d'arrivée (32) se trouvant dans l'un des couvercles (31, 31.1) et une sortie (33) dans l'autre couvercle, et que la section du canal (29.1) du bloc (28.1), fermé par le couvercle (31.1) comportant une sortie (33), est de dimension plus petite que la section du canal (29) de l'autre bloc (28).

2. Évaporateur selon la revendication 1, **caractérisé en ce que** l'élément chauffant PTC (27) est maintenu sous précontrainte entre les blocs (28, 28.1) par ses côtés plats.

3. Évaporateur selon la revendication 1 ou 2, **caractérisé en ce que** chaque bloc (28, 28.1) présente un canal de liaison (30, 30.1), lesquels sont reliés l'un à l'autre par une douille d'étanchéité D.

4. Procédé pour mettre à disposition un flux homogène de vapeur à l'aide d'un évaporateur selon l'une des revendications 1 à 3, **caractérisé en ce qu'**une arrivée d'eau jusqu'à l'évaporateur (26) se fait de façon pulsée.

5. Procédé selon la revendication 4, **caractérisé en ce que** l'arrivée d'eau pulsée s'effectue en fonction de la température actuelle de l'élément chauffant PTC (27).
